# FASCICULE DE BREVET EUROPEEN

(11) **EP 3 253 334 B1**
(45) Date de publication et mention de la délivrance du brevet: **03.04.2019**
(21) Numéro de dépôt: 16703126.9
(22) Date de dépôt: 05.02.2016
(51) Int. Cl.: A61F 2/38

(54) **PROTHESE TOTALE DE GENOU AVEC COUPLE DE FROTTEMENT CERAMIQUE SUR CERAMIQUE ET PLATEAU MOBILE EN CERAMIQUE**
TOTALKNIEPROTHESE MIT KERAMIK-AUF-KERAMIK-REIBUNGSMOMENT UND MOBILE KERAMIKPLATTE
TOTAL KNEE PROSTHESIS WITH CERAMIC ON CERAMIC FRICTION TORQUE AND MOBILE CERAMIC PLATE

(30) Priorité: 05.02.2015 FR 1550908
(43) Date de publication de la demande: 13.12.2017
(73) Titulaire: Assistance Publique - Hôpitaux De Paris, 75475 Paris Cedex 10 (FR)
(72) Inventeur: SEDEL, Laurent, 78350 Jouy en Josas (FR)
(74) Mandataire: Vidon Brevets & Stratégie
(86) Numéro de dépôt international: PCT/EP2016/052486
(87) Numéro de publication internationale: WO 2016/124731

(56) Documents cités:
- WO-A1-2010/001010
- WO-A2-2011/003621
- GB-A- 2 341 803
- US-A- 5 064 437

## Description

### 1. Domaine de l'invention

Le domaine de l'invention est celui de la prothèse articulaire.

Plus précisément, l'invention concerne une prothèse totale en céramique comprenant un plateau mobile en céramique pour remplacer l'articulation du genou chez un patient.

### 2. Art antérieur

Le remplacement de l'articulation du genou est une intervention fréquente. On estime qu'en 2011, environ 650 000 prothèses ont été posées aux Etats-Unis et 80 000 en France. Les prothèses du genou peuvent être totales ou partielles : unicondyliennes, totales et alors à charnière ou à glissement.

Les prothèses à glissement comprennent une pièce fémorale condylienne et une embase tibiale, fixées respectivement sur les extrémités profilées du fémur et du tibia. La majorité des prothèses posées sont des prothèses dites « à glissement ». Ces prothèses à glissement comportent une pièce fémorale en métal ou en céramique et une pièce tibiale en polyéthylène généralement encastrée dans l'embase tibiale en métal. Certaines présentent un plateau mobile en polyéthylène qui peut se déplacer sur le plateau tibial. L'avantage théorique du plateau mobile est de diminuer l'usure du polyéthylène en permettant une adaptation plus cohérente des surfaces de frottement et d'améliorer la flexion du genou lorsque le déplacement du plateau mobile se fait non seulement en rotation mais aussi d'avant en arrière (mouvement de « tiroir »), le plateau mobile se déplaçant sur la surface tibiale à la manière du ménisque.

Ces prothèses à glissement ne remplacent pas les ligaments croisés du genou et doivent donc être posées en respectant parfaitement l'architecture naturelle des surfaces du genou et des axes anatomiques. Certaines conservent le ligament croisé postérieur, d'autres non, il existe alors un capot central qui évite la chute du tibia en arrière.

Jusqu'à présent, les prothèses totales sont réalisées en métal et en polyéthylène. Selon cette technique, les embases fémorales et tibiales sont réalisées en un matériau métallique, tandis qu'un tampon de polyéthylène est monté de manière fixe ou mobile sur l'embase tibiale. D'autres modes de réalisation consistent en une embase fémorale en céramique coopérant avec un plateau en polyéthylène, monté sur une embase tibiale en céramique ou en métal. Lorsque la prothèse est conçue avec un plateau mobile, la mobilité du plateau mobile peut être limitée par la forme du réceptacle métallique fixé dans le tibia qui peut n'autoriser que des mouvements de rotation ou par un téton central qui permet selon la forme d'une cavité inférieure dessinée dans le plateau mobile des mouvements de rotation et de tiroir.

Toutefois, ces matériaux ne permettent pas de concevoir une prothèse articulaire suffisamment robuste pour permettre aux patients de reprendre une activité physique normale: s'il est aisé de marcher à nouveau sans douleur avec une prothèse du genou, les patients ressentent une gêne lors d'une marche prolongée ou à la montée et la descente des escaliers. La course ou toute autre activité physique importante restent par ailleurs interdites.

Or, l'âge moyen des patients diminue considérablement. De nombreuses interventions font en effet suite à un accident du sport ou du travail, chez des personnes jeunes et professionnellement actives. Il est par conséquent difficile et particulièrement frustrant pour ces personnes de devoir renoncer à toute forme d'activité physique, voire à leur activité professionnelle.

De plus, le taux d'échec de ces prothèses est actuellement de 10% au bout des dix premières années. Ce taux augmente à 25% chez les patients les plus jeunes. L'échec se traduit par la nécessité de réopérer le patient afin de remplacer sa prothèse. Or, ces ré-interventions impliquent d'une part une souffrance importante pour le patient, et d'autre part un coût conséquent pour les systèmes d'assurance maladie. Elles représentent également un risque juridique pour les institutions cliniques ou hospitalières.

Mais la cause principale de ces échecs réside principalement dans le phénomène d'usure du polyéthylène constituant du couple de frottement. La libération sous contraintes mécaniques des particules d'usure engendre une réaction inflammatoire responsable d'épanchement articulaire, mais aussi de la création d'un granulome de résorption périarticulaire. Une lyse osseuse au niveau des parties du fémur et/ou du tibia avoisinant la prothèse a été observée, pouvant résulter en une perte osseuse importante. Cette ostéolyse peut également conduire au descellement de la prothèse. Lorsque l'usure a été très importante, les pièces métalliques peuvent se trouver mises à nu, aboutissant à des réactions de rejet massives par production de particules métalliques toxiques (metallose).

On connaît le document WO-2011/003621-A1 qui décrit notamment des prothèses de genou partiellement en céramique ainsi qu'un dispositif d'usure de ces prothèses. Toutefois, aucune prothèse selon cette technique n'est à ce jour apparue sur le marché, et ce, pour les raisons suivantes.

Tout d'abord, les prothèses selon ce document comprennent une tige en métal sur laquelle est fixée l'embase tibiale, ce qui induit une forte abrasion du métal. L'abrasion du métal se traduit par la formation de débris métallique dans le corps. L'organisme réagit contre ces particules de métal en produisant une réaction inflammatoire comme décrit avec les particules de polyéthylène.

De plus, la fixation des pièces en céramique sur une tige métallique est problématique : il est dans la pratique très difficile de fixer ce type de pièces sans créer des conditions de rupture de la céramique. Le document WO 2011/003621 A1 ne décrit d'ailleurs aucune méthode de fixation de ces pièces. En outre, bien que le plateau mobile soit décrit en céramique, la large ouverture ovale au centre du plateau mobile est dans la pratique impossible à réaliser dans un matériau en céramique massive, cette forme d'ouverture et ses dimensions fragilisant la céramique. De même, le choc de l'ouverture pratiquée dans le plateau en céramique contre les butées 18 est de nature à induire des fractures prématurées du plateau en céramique, s'il était en céramique massive.

Une solution pour contrer ces inconvénients est de proposer des pièces en métal recouvertes de céramique. Toutefois, la fine couche de céramique à la surface du métal s'use et le métal est peut être mis à nu, provoquant les réactions inflammatoires précédemment évoquées. Cette pratique n'a pas pour le moment montré une quelconque supériorité.

Il est donc nécessaire de concevoir des prothèses totales du genou qui soient plus résistantes à l'usure, que les particules d'usure produites ne génèrent pas de réactions inflammatoires et qui permettent aux patients de retrouver une vie normale. Il est en outre souhaitable que la prothèse ou ses débris produisent un tissu fibreux dense susceptible de stabiliser le genou comme le feraient les ligaments.

### 3. Objectifs de l'invention

L'invention a notamment pour objectif de pallier ces inconvénients de l'art antérieur.

Plus précisément, un objectif de l'invention est de fournir, dans au moins un mode de réalisation, une prothèse de genou qui soit plus résistante que les prothèses actuelles.

Un autre objectif de l'invention est de mettre en oeuvre, dans au moins un mode de réalisation, une telle prothèse qui permette aux patients de reprendre une activité physique normale.

L'invention a encore pour objectif de proposer, dans au moins un mode de réalisation, une prothèse qui limite les réactions inflammatoires.

L'invention a encore pour objectif, dans au moins un mode de réalisation, de proposer une prothèse qui permette de reformer un tissu fibreux plus résistant jouant le rôle naturel des ligaments du genou par remodelage sous contrainte mécanique des structures fibreuses.

L'invention a encore pour objectif, dans au moins un mode de réalisation, de proposer une prothèse dont les surfaces articulaires (surfaces de frottements mutuels) présentent une congruence améliorée en zone de charge.

L'invention a encore pour objectif, dans au moins un mode de réalisation, de proposer une prothèse permettant une plus grande amplitude de mouvement, en comparaison avec les prothèses de genou connues.

### 4. Exposé de l'invention

Ces objectifs, ainsi que d'autres qui apparaîtront par la suite, sont atteints à l'aide d'une prothèse de genou totale destinée à être implantée chez un patient humain comme définie dans la revendication 1.

Cette distance permet une congruence parfaite entre les surfaces de frottements mutuels notamment en zone de charge (par exemple en position debout statique ou en mouvement).

Dans la description de l'invention qui va suivre, l'axe longitudinal de l'élément fémoral est essentiellement confondu avec l'axe du fémur du patient chez qui la prothèse sera implantée. De même, l'axe longitudinal du plateau tibial est essentiellement confondu avec l'axe longitudinal du tibia du patient recevant la prothèse. Par « essentiellement confondu », on entend que les deux axes de référence forment un angle inférieur à 5° entre eux, alternativement inférieur à 2°, alternativement inférieur 1°, alternativement forment un angle égal à environ 0°.

Ainsi, la prothèse selon l'invention comprend donc trois pièces en céramique d'alumine massive destinée à s'articuler les unes avec les autres selon deux articulations à surfaces congruentes. L'élément fémoral vient s'emboiter sur l'extrémité distale préparée du fémur, et reproduit la forme de la trochlée. Il en faut donc un droit et un gauche. Le plateau tibial est ancré dans l'extrémité supérieure du tibia préparé à le recevoir. Enfin, le plateau mobile est situé entre l'élément fémoral et le plateau tibial pour former une articulation. L'invention repose donc sur une approche tout à fait nouvelle et originale de concevoir une prothèse totale de genou bicondylienne dans laquelle toutes les surfaces en contact des éléments la composant sont en un même matériau céramique. Ceci favorise une réduction de la quantité de débris d'usure dans la mesure où le dessin des pièces avec deux articulations distinctes permet de conserver dans tous les degrés de flexion du genou un contact important entre les surfaces, élément important pour réduire la quantité de débris formé. Ce contact ou congruence est obtenu tant en zone de charge, qu'en zone de flexion.

Au contraire des prothèses actuelles qui comprennent un élément fémoral en métal ou en céramique, en contact avec un plateau fixe ou mobile en polyéthylène, la prothèse selon l'invention comprend un plateau mobile en un même matériau céramique massif que l'élément fémoral.

Selon l'invention, l'élément fémoral, le plateau mobile et le plateau tibial sont entièrement constitués dans un même matériau céramique. En d'autres termes, l'élément fémoral, le plateau mobile et le plateau tibial sont tous les trois réalisés en céramique massive ce qui signifie qu'ils ne comprennent pas d'alliage avec un matériau plastique ou métallique tel le polyéthylène, le titane, la céramique métallisée, etc. ou qu'il ne s'agit pas d'une fine couche de céramique fixée sur un élément en métal ou en plastique.

Jusqu'à présent, il n'avait jamais été proposé, à la connaissance de la Déposante, de mettre en contact étroit deux pièces en céramique pour concevoir une prothèse du genou. Il était connu de proposer des prothèses en céramique pour la hanche, chez les personnes jeunes et /ou actives. Toutefois, les sollicitations mécaniques exercées sur l'articulation du genou sont très différentes de celles exercées au niveau de la hanche, les articulations de la hanche et du genou ayant des géométries différentes. L'utilisation de la céramique pour la réalisation d'une prothèse de genou dans laquelle les surfaces de frottements du plateau tibial et de l'élément fémoral sont entièrement en céramique a toujours été rejetée par les prothésistes et les chirurgiens orthopédiques, et ce, pour différentes raisons. La première de ces raisons est que la mécanique du genou est complexe. Lors de la flexion, il se produit des phases successives de roulement et de glissement. Ces phases sont bien tolérées par un couple de matériaux dur/mou, mais ne peuvent être envisageables avec un couple dur/dur, puisque ce dernier ne fonctionne de façon tribologique satisfaisante que s'il persiste une congruence importante entre les surfaces de frottement.

La deuxième est que la céramique est un matériau de comportement fragile qui peut se casser lors de la propagation d'une fissure.

Outre le risque sanitaire pour le patient, les cas de rupture de prothèse représentent un coût et un risque juridique important pour les professionnels de santé. Il est à comparer aux risques 10 fois plus élevés de ré-interventions dues aux conséquences des réactions aux débris d'usure des prothèses contenant du polyéthylène.

Un autre préjugé à l'encontre de l'utilisation de la céramique dans le cadre d'une prothèse totale pour genou est la possibilité que le frottement de deux pièces en céramique l'une contre l'autre produise un crissement à chaque mouvement, ce bruit étant entendu par le patient et parfois même par l'entourage du patient. Ce phénomène a déjà été observé dans certaines prothèses de la hanche et peut être gênant au quotidien pour le patient. Le dessin des pièces ainsi que l'interposition de particules métalliques est l'élément en cause les plus souvent rapporté. L'invention permet d'éviter cet inconvénient en prévoyant une épaisseur de céramique suffisamment épaisse et en supprimant les surfaces de frottements mutuels céramique/plastique ou céramique/métal. Par ailleurs, les pièces de la prothèse selon l'invention peuvent être fixées dans l'os par l'intermédiaire d'un ciment acrylique qui permet d'amortir les vibrations.

De préférence, ledit matériau céramique présente une épaisseur comprise entre 4 mm et 14 mm. La Déposante a en effet constaté que les prothèses en céramique dont l'épaisseur se situe en dehors de cette plage sont cassantes, ce qui n'est pas souhaitable. En particulier, il a été proposé comme prototype des prothèses dans lesquelles une fine couche de céramique était fixée sur une partie métallique. De telles prothèses sont extrêmement cassantes et ne sont donc pas exploitées sous forme de frottements céramique/céramique.

Un autre avantage de la céramique est qu'elle est davantage tolérée par l'organisme que le métal ou le polyéthylène. L'avantage du frottement céramique contre céramique est de permettre de recréer un tissu fibreux ou « néoligament » jouant le rôle des ligaments naturels après remodelage adaptatif. On observe en effet la formation d'un tissu fibreux plus résistant et de meilleure qualité avec les prothèses selon l'invention qu'avec des prothèses usuelles dans lesquelles les surfaces de frottements sont en métal contre du polyéthylène ou en céramique contre du polyéthylène. Les particules d'usure, générées en faible quantité lors des mouvements de l'articulation, ne provoquent pas de réaction macrophagique dans l'organisme. Par conséquent, les prothèses selon l'invention permettent d'éviter le problème d'ostéolyse et le risque de descellement de la prothèse observés avec les prothèses actuelles.

Bien au contraire, la Déposante constate que de faibles quantités de particules d'alumine permettent de générer un tissu fibreux particulièrement dense, plus dense que celui formé autour des prothèses actuelles. Ce tissu fibreux participe à renforcer et stabiliser la capsule articulaire et à remplacer les ligaments. Par conséquent, les prothèses selon l'invention permettent aux patients de retrouver une articulation du genou plus stable et plus résistante à l'effort. Cette particularité permet donc aux patients de conserver leur prothèse plus longtemps et de recouvrer une activité physique normale.

La Déposante a, en outre, constaté que le comportement de deux pièces en céramique l'une contre l'autre permettait d'obtenir une prothèse très résistante et plus robuste que celles existantes, contrairement aux préjugés énumérés ci-dessus. Ces avantages techniques particuliers sont dus notamment à la parfaite congruence entre les surfaces de frottements ce qui permet de diminuer les frictions entre les pièces et par conséquent, le risque d'usure de la prothèse.

Au sens de l'invention et dans la description qui suit, la parfaite congruence des pièces les unes par rapport aux autres est obtenue lorsque les pièces se conforment mutuellement de manière parfaite, c'est-à-dire avec des jeux très réduits entre les surfaces en contact, et lorsqu'il n'existe aucune zone au niveau des surfaces de frottement des pièces l'une contre l'autre dans laquelle les contraintes mécaniques sont concentrées. Cette congruence parfaite peut également être qualifiée d'idéale dans la description.

Cette congruence parfaite ou idéale des surface de frottements mutuels est obtenue grâce aux choix de formes simples des surfaces : les surfaces condyles/cuvettes condyliennes sont des portions de cylindres et/ou de sphère de même rayon de courbure et la surface de contact plateau mobile/plateau tibial est plane. Ces formes sont obtenues par des techniques de fabrication et de rodage spécifiques, maîtrisées par l'industrie spécialisée en prothèses céramiques. Brièvement, les profils des différentes pièces produites sont évalués grâce à la mesure des rayons de courbure des différentes pièces après avoir soumis les pièces à différents tests d'usure. L'objectif de ces mesures est d'obtenir un espacement entre les pièces, et plus précisément entre le plateau mobile et l'élément fémoral.

Selon l'invention, l'espace entre les surfaces de frottements mutuelles de l'élément fémoral et du plateau mobile est compris entre 0 µm et 100 µm, de préférence entre 10 µm et 60 µm, lorsque les axes longitudinaux (L, L') dudit élément fémoral et dudit plateau tibial forment un angle compris entre 0° et 75° avec l'axe longitudinal du plateau mobile.

Avantageusement, l'espace compris entre les surfaces de frottements mutuels du plateau mobile et du plateau tibial est également compris entre 0 µm et 100 µm, de préférence entre 10 et 100 µm, de manière davantage préférée entre 10 µm et 60 µm.

La sélection de la distance entre les surfaces de frottements mutuels est de la première importance. En effet, les inventeurs ont démontré de manière surprenante que cette distance, afin d'assurer une congruence optimale sans altérer le confort et le fonctionnement de l'articulation prothétique, ne devrait de préférence pas être en deçà de 10 µm et au delà de 100 µm, de préférence pas au delà de 60 µm. En effet, en dessous de 10 µm, le liquide risque de ne pas circuler suffisamment. Au delà de 100 µm, le risque de concentration des contraintes sur une partie de la prothèse devient trop élevé.

Pour toutes ces raisons, les prothèses selon l'invention sont plus résistantes et limitent, voire évitent, les inconvénients observés avec les prothèses actuelles dans lesquelles le plateau mobile est en polyéthylène. La présente invention permet ainsi aux patients de retrouver une vie normale avec un niveau d'activité physique supérieur par rapport à ce qui leur est aujourd'hui permis. Il n'est plus nécessaire de les réopérer pour remplacer la prothèse usagée ou, à tout le moins, l'échéance d'une nouvelle opération est considérablement retardée par rapport aux prothèses actuelles.

Jusqu'à présent, il était considéré comme impossible de concevoir une prothèse totale du genou dans laquelle les éléments formant l'articulation - c'est à dire l'élément fémoral, le plateau mobile et le plateau tibial - étaient entièrement en céramique massive en raison du fait qu'aucun dessin adéquat n'a été proposé. Il n'était en effet pas possible, avec les dessins existants, de conserver une congruence parfaite lors de l'entièreté du mouvement, cette congruence étant indispensable lorsqu'on souhaite faire frotter de la céramique sur elle-même. Le dessin particulier de la prothèse selon l'invention autour d'un plateau mobile de forme spécifique et l'espacement prévu entre les surfaces ensembles permettent de supprimer ces zones de concentration des contraintes mécaniques, et par conséquent permettent de fabriquer des prothèses totales de genou dans laquelle tous les éléments formant l'articulation sont en céramique massive.

Il s'agit donc ici d'une sélection de caractéristiques particulières et spécifiques qui permettent de concevoir et mettre en oeuvre une prothèse totale du genou en céramique.

De préférence, le matériau céramique est une céramique d'alumine (Al₂O₃), qui permet d'obtenir la synthèse d'un tissu fibreux de meilleure qualité par rapport aux autres céramiques. De tels matériaux sont de qualité chirurgicale, c'est à dire qu'ils doivent être conformes aux normes en vigueur relatives aux matériaux pour la fabrication de prothèses chirurgicales. Par exemple, la céramique d'alumine peut être une céramique dense polycristalline obtenue à partir d'une poudre d'oxyde d'aluminium compressée à des températures d'environ 1600°C grâce au procédé HIP (High Isostatic Pressure). Une céramique convenant à la mise en oeuvre de l'invention est une céramique conforme à la norme ISO-6474-1.

De préférence, la céramique présente une granulométrie inférieure à 2 µm. De manière davantage préférée, la céramique utilisée pour la fabrication de la prothèse selon l'invention présente une granulométrie comprise entre 0,5 µm et 2 µm. De manière encore davantage préférée, la céramique a une granulométrie 1 µm et 2 µm. La granulométrie du matériau céramique est déterminée selon toute méthode bien connue de l'homme de l'art.

Dans une variante intéressante de l'invention, l'élément fémoral est également apte à coopérer avec la rotule du patient, via une protubérance mimant une trochlée sur la partie antérieure de l'élément fémoral.

Avantageusement, le plateau tibial est ancré par sa face inférieure dans l'extrémité supérieure du tibia préparé d'un patient. Un dessin en forme de tige massive et faisant corps avec le plateau tibial est fixée dans l'os du patient par du ciment acrylique.

Les dimensions des pièces constituant la prothèse sont fonction de la taille et des dimensions de l'articulation à remplacer. Par exemple, le plateau mobile peut présenter une épaisseur maximale comprise entre 4 mm à 14 mm. Cette épaisseur de céramique est suffisante pour assurer la stabilité du genou tout en évitant le risque de fracture de la céramique.

Avantageusement, lesdites cuvettes condyliennes et lesdits condyles correspondants sont espacées l'un de l'autre d'une distance inférieure à 100 µm, de préférence une distance comprise entre 10 µm et 100 µm et de manière davantage préférée une distance comprise entre 10 µm et 60 µm, lorsque l'axe longitudinal de l'élément fémoral et l'axe longitudinal du plateau tibial forme un angle compris entre 0° et 60°. La plage angulaire comprise entre 0° et 60° correspond à la zone de charge de la prothèse. La zone de charge est définie par la zone et l'amplitude de la flexion du genou sur lesquelles l'articulation supporte l'essentiel du poids du patient.

Avantageusement, l'élément fémoral et le plateau tibial sont mobiles en rotation l'un part rapport à l'autre sur une plage angulaire de flexion comprise entre 0° et 135°.

En d'autres termes, l'axe longitudinal du plateau tibial et l'axe longitudinal de l'élément fémoral peuvent former un angle compris entre 0° et 135°.

Au sens de l'invention, le plateau mobile est mobile dans une direction avant/arrière, latérale et/ou pivotante autour d'un axe longitudinal. Ainsi, le plateau mobile selon l'invention n'est jamais uniquement rotatoire.

Cette caractéristique permet au patient recevant la prothèse d'être davantage libre de ses mouvements et notamment de pouvoir monter un escalier, plier le genou pour s'accroupir ou s'agenouiller, ou simplement avoir davantage d'amplitude dans ses mouvements sans ressentir de blocage. Les mouvements permis par la prothèse de genou selon l'invention sont alors très proches des mouvements naturels permis par le genou. C'est le mouvement propre du genou de chaque patient qui crée la position des zones de tissus fibreux néoformés et permettent ainsi après quelques semaines d'obtenir le résultat escompté. Cette amplitude de mouvement par exemple n'est pas permise par les prothèses décrites dans le document WO-2011/003621-A1 dans lequel les prothèses ne peuvent plier au delà de 90°, ce qui interdit au patient de monter un escalier par exemple, et fait donc des prothèses décrites dans ce document des prothèses impossibles à proposer à un patient.

Dans un mode de réalisation intéressant, les surfaces de frottements mutuels des condyles et des cuvettes condyliennes sont des portions de cylindre de révolution ayant même rayon R.

Dans un autre mode de réalisation, les surfaces de frottements mutuels des condyles et des cuvettes condyliennes sont des portions de sphère ayant même rayon R'.

Dans un autre mode de réalisation, l'une des surfaces de frottements mutuels des condyles et des cuvettes condyliennes est une portion de sphère ayant un rayon R' et l'autre est une portion de cylindre de révolution ayant un rayon R, le centre de la sphère étant sur l'axe de révolution du cylindre.

La forme de portion de cylindre et/ou de sphère s'apprécie lorsqu'on regarde une coupe sagittale de la prothèse, réalisée à environ 20 à 27 mm du plan médian de la prothèse selon l'invention. L'homme du métier sait déterminer aisément comment placer le plan sagittal.

Il est à noter que les condyles naturels ont généralement une forme sphérique faite de plusieurs sphères successives dont les rayons diminuent d'avant en arrière, le centre des sphères ayant une forme d'ellipse. La Déposante propose ici une forme légèrement différente de la forme naturelle permettant d'assurer la congruence idéale des éléments de la prothèse les uns par rapport aux autres lors du mouvement du genou, et donc leur réalisation en céramique. En effet, les centres des portions de cylindre ou de sphère des surfaces de frottement entre le plateau mobile et l'élément fémoral se trouvent non pas sur une ellipse mais sur un axe.

Dans des variantes, R et R' peuvent être égaux. Les dimensions des prothèses et des pièces la constituant sont aisément déterminées par l'homme du métier. Il est en effet courant de proposer différentes tailles de prothèses pour s'adapter aux différences de taille du patient opéré.

Ces trois modes de réalisation permettent de concevoir une prothèse dans laquelle les contraintes mécaniques ne sont pas concentrées sur une zone précise ce qui pourrait induire une fissure de la céramique.

Avantageusement, ledit plateau mobile comprend un plot intercondylien formant saillie en direction dudit élément fémoral, ledit l'élément fémoral comprenant un vide intercondylien logeant ledit plot intercondylien. Ainsi, l'élément fémoral et le plateau mobile coopèrent le long d'une troisième série de surfaces de frottements mutuels. De préférence, le vide intercondylien et le plot intercondylien sont des portions de cylindre ou des portions de sphère. Si le plot intercondylien et le vide intercondylien sont des portions de cylindre, elles sont coaxiales des portions de cylindre formant les condyles et les cuvettes condyliennes. Si le plot intercondylien et le vide intercondylien sont des portions de sphère, le centre de cette sphère se trouve :
- soit sur l'axe de révolution des portions de cylindre formant les condyles et les cuvettes condyliennes ;
- soit sur l'axe reliant les centres des portions de sphère formant les condyles et les cuvettes condyliennes.

Le plot intercondylien et le vide intercondylien ensemble permettent d'apporter à la fois de la stabilité et de centrer le mouvement relatif de l'élément fémoral et du plateau mobile. Le plot intercondylien bute sur les extrémités de la fente ce qui permet de limiter l'amplitude du mouvement lors de l'extension.

Le rayon R" de la portion de sphère ou de cylindre forment le vide intercondylien peut être compris entre 14 mm et 30 mm, de préférence entre 17 mm et 25 mm.

Il est à noter que les rayons R, R' et R" se mesurent entre l'axe du cylindre de révolution ou le centre de la sphère, et la surface de frottement d'une pièce contre l'autre.

Avantageusement, lesdits vide et plot condyliens sont espacées l'un de l'autre d'une distance comprise entre 0 µm et 100 µm, de préférence entre 10 µm et 100 µm, de manière davantage préférée entre 10 µm et 60 µm lorsque l'axe longitudinal de l'élément fémoral et l'axe longitudinal du plateau mobile forme un angle compris entre 0° et 60°.

Avantageusement, R et R' ont une longueur comprise entre 22 mm et 38 mm, de préférence entre 25 mm et 35 mm.

La Déposante a constaté que ces dimensions particulières permettaient de concevoir des prothèses de genou pour des individus de corpulence différente tout en évitant la concentration des contraintes sur une zone précise du matériau céramique. Autrement dit, ces dimensions particulières permettent d'obtenir la congruence parfaite nécessaire pour réaliser une prothèse selon l'invention, dans laquelle élément fémoral, plateau mobile et plateau tibial sont en céramique massive.

Avantageusement, ledit plateau mobile présente un périmètre inférieur au périmètre de la surface supérieure du plateau tibial. Cette caractéristique permet à la prothèse d'avoir un mouvement légèrement pivotant autour de l'axe longitudinal du plateau mobile et du plateau tibial, axe longitudinal essentiellement confondu avec l'axe longitudinal du tibia. Il est à noter que l'axe longitudinal du plateau mobile est essentiellement parallèle, de préférence parfaitement parallèle, à l'axe longitudinal du plateau tibial.

Dans un mode de réalisation avantageux, ledit plateau tibial comprend au moins trois butées formant saillie en direction dudit plateau mobile, lesdites butées permettant de limiter les mouvements du plateau mobile à la surface du plateau tibial.

Les butées permettent d'éviter une translation incontrôlée du plateau mobile à la surface du plateau tibial. Les butées définissent donc un périmètre de mouvement libre mais contrôlé du plateau mobile par rapport au plateau tibial. Ainsi, les mouvements autorisés par la prothèse sont très proches des mouvements d'un genou naturel.

Avantageusement, la surface de frottements mutuels entre ledit plateau mobile et ledit plateau tibial est essentiellement plane. De préférence, la surface de frottements mutuels entre ledit plateau mobile et ledit plateau tibial est parfaitement plane. Ainsi, les mouvements de glissement et/ou de translation des plateaux l'un par rapport à l'autre sont quasiment ou complètement dépourvus de friction. Sans aspérité, les risques d'usure sont limités. Le mouvement du genou est donc plus fluide et plus naturel.

Dans un mode de réalisation avantageux, ledit plateau mobile peut pivoter sur ledit plateau tibial autour d'un axe selon un angle de +/- 15°. En d'autres termes, l'axe transversal du plateau mobile peut former un angle de 15° par rapport à l'axe transversal du plateau tibial. On note par convention un pivotement de +15° lorsque le mouvement se fait dans le sens des aiguilles d'une montre et de -15° lorsque le mouvement se fait dans le sens contraire des aiguilles d'une montre. Cette amplitude de mouvement est suffisante pour assurer les mouvements de rotation d'un genou naturel, en particulier lors de la flexion du genou.

Dans un mode de réalisation avantageux, les pièces formant la prothèse selon l'invention sont dépourvues d'orifice et/ou de perforations. Ainsi, l'élément fémoral, le plateau tibial et le plateau mobile sont dépourvus d'orifice et de perforations. Considérant que ces pièces sont constituées dans un même matériau céramique massif, il s'ensuit que l'intégrité de la céramique est préservée, en n'y ménageant aucun orifice et aucune perforation. Cela contribue davantage à la résistance de la prothèse.

Dans un mode de réalisation avantageux, chaque pièce formant la prothèse est une pièce unitaire. Ainsi, l'élément fémoral, le plateau tibial et le plateau mobile sont chacun formé d'une pièce unique en un matériau céramique massif. A contrario, aucun d'entre eux ne comprend de pièce secondaire additionnelle. De même, aucun d'entre eux n'est formé par l'assemblage d'au moins deux pièces distinctes. Cela contribue davantage à la résistance de la prothèse et à la simplicité de conception.

### 5. Liste des figures

D'autres caractéristiques et avantages de l'invention apparaîtront plus clairement à la lecture de la description suivante d'un mode de réalisation préférentiel, donné à titre de simple exemple illustratif et non limitatif, et des dessins annexés, parmi lesquels :
- la figure 1 présente une vue en perspective et en éclaté de la prothèse selon l'invention ;
- la figure 2 est une coupe sagittale de la prothèse selon l'invention lorsque l'axe longitudinal de l'élément fémoral et l'axe longitudinal du plateau mobile forme un angle nul (0°) ;
- la figure 3 est une coupe sagittale de la prothèse selon l'invention lorsque l'axe longitudinal de l'élément fémoral et l'axe longitudinal du plateau mobile forme un angle de 45° ;
- la figure 4 est une coupe sagittale de la prothèse selon l'invention lorsque l'axe longitudinal de l'élément fémoral et l'axe longitudinal du plateau mobile forme un angle de 135° ;
- la figure 5A est une vue de dessus du plateau tibial et du plateau mobile lorsque l'axe transversal du plateau mobile et l'axe transversal du plateau tibial forme un angle de +15°;
- la figure 5B est une vue de dessus du plateau tibial et du plateau mobile lorsque l'axe transversal du plateau mobile et l'axe transversal du plateau tibial forme un angle de -15°;
- la figure 5C est une vue de dessus du plateau tibial et du plateau mobile lorsque l'axe transversal du plateau mobile et l'axe transversal du plateau tibial forme un angle nul (0°).

### 6. Description d'un mode de réalisation de l'invention

Le principe général de l'invention repose sur une prothèse totale pour genou dans laquelle le plateau mobile, l'élément fémoral et le plateau tibial ont leurs surfaces de frottements mutuels en un même matériau céramique. Ces surfaces de frottements mutuels sont parfaitement congruentes grâce à l'optimisation de la forme des surfaces de frottements mutuels et la distance respective entre les pièces. Contrairement à l'idée couramment répandue jusqu'à présent, la céramique permet d'obtenir une prothèse pour genou particulièrement résistante.

Cette résistance est également due à la parfaite congruence des pièces les unes par rapport aux autres, et notamment du plateau mobile par rapport à l'élément fémoral et du plateau mobile par rapport au plateau tibial. La parfaite congruence du plateau mobile avec l'élément fémoral assure un frottement optimal tout en générant un minimum de débris d'usure, sa mobilité assure la reproduction du jeu articulaire global décomposé en deux mouvements : celui de l'élément fémoral sur le plateau mobile associé à la mobilité rotatoire et en tiroir du plateau mobile sur la surface de l'embase tibiale. L'ensemble de l'innovation permet donc d'éviter, ou à tout le moins de limiter, les probabilités de ré-opération du genou, en particulier chez les patients jeunes et physiquement actifs. Elle permet en outre d'améliorer les résultats de la prothèse du genou en termes de confort et de stabilité. Cette parfaite congruence est obtenue grâce aux travaux des inventeurs, qui ont conduit à développer des surfaces de frottements mutuels qui permettent d'éviter la création de concentration de contraintes mécaniques dans la prothèse. La congruence permet donc d'éviter le phénomène d'usure anormale du matériau céramique.

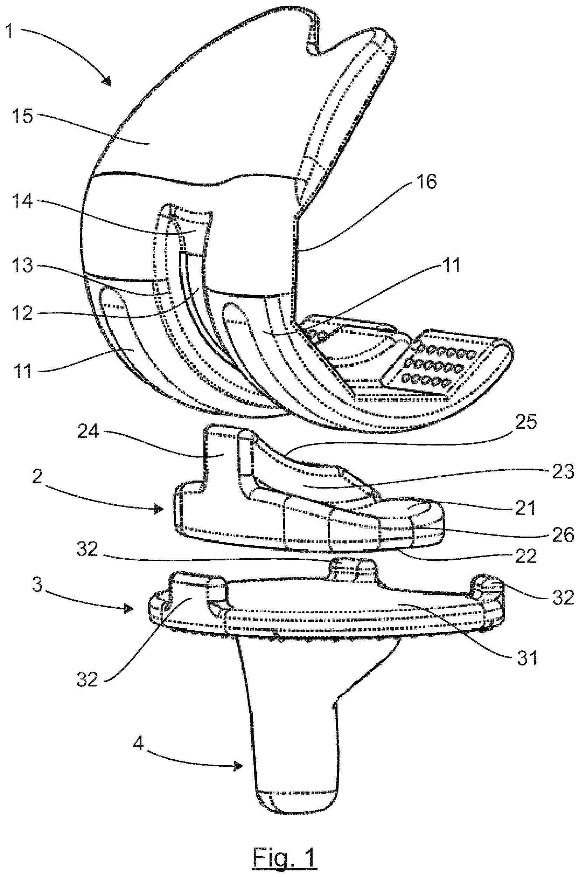

On présente, en relation avec la figure 1, une vue en perspective et en éclaté de la prothèse selon la présente invention.

Comme on peut le voir sur la figure 1, la prothèse selon l'invention comprend un élément fémoral 1, un plateau mobile 2 et un plateau tibial 3 fixé sur un talon d'ancrage 4 destiné à être monté sur une extrémité supérieure profilée d'un tibia.

L'élément fémoral 1 se présente sous la forme générale d'une coque partielle creuse définissant extérieurement deux condyles 11 reproduisant sensiblement la forme des condyles naturels, mais dont la partie postérieure flottante avec le plateau mobile sont des portions de cylindres et non des portions de sphères irrégulières. Les condyles 11 pourraient également être des portions de sphère. Les condyles 11 sont séparés par une fente qui reproduit globalement le vide intercondylien 12 naturel. Le vide intercondylien 12 présente deux surfaces latérales sensiblement parallèles 13. Le vide intercondylien 12 correspond à une portion de cylindre coaxiale aux portions de cylindre formant les condyles 11, mais de rayon inférieur. Le vide intercondylien 12 peut également prendre la forme d'une portion de sphère dont le centre se trouve sur l'axe de révolution des portions de cylindre formant les condyles 11 ou sur l'axe reliant le centre des sphères lorsque les condyles sont des portions de sphère.

Intérieurement, l'élément fémoral définit un logement fémoral 16 destiné à recevoir l'extrémité inférieure profilée F1 d'un fémur F. L'élément fémoral 1 peut présenter sur sa face antérieure 15 une protubérance mimant la trochlée et destinée à coopérer avec la rotule du patient.

Le plateau mobile 2 est inséré entre l'élément fémoral 1 et le plateau tibial 3. Le plateau mobile présente une surface inférieure 23 essentiellement, voire parfaitement, plane. A sa surface supérieure, le plateau mobile 2 présente deux cuvettes condyliennes 21 séparées par un plot intercondylien 24. Le plot intercondylien présente une surface 25 dont la forme correspond au vide 12 ainsi que deux surfaces latérales 23 dont la forme correspond aux surfaces latérales 13 du vide. Un léger interstice permet aux liquides naturels de lubrifier l'articulation formée par la prothèse.

Les cuvettes condyliennes 21 définissent des zones ou surfaces de frottements mutuels, respectivement pour les deux condyles 11 de l'élément fémoral 1. Avantageusement, les cuvettes 31 et les condyles 11 présentent des surfaces de frottements mutuels de forme cylindrique parfaitement congruentes. Cela réduit significativement les frottements, et de ce fait l'usure de ces pièces. On comprend par « parfaitement congruente » que les pièces s'emboitent parfaitement entre elles, qu'elles sont espacées d'une distance comprise entre environ 0 µm et 100 µm, de préférence entre 10 µm et 100 µm, de manière davantage préférée entre 10 µm et 60 µm, notamment lorsque l'angle entre les axes longitudinaux de l'élément fémoral et du plateau mobile forme un angle compris entre 0° et 75°.

Quant au plot intercondylien 24, il est engagé dans le vide intercondylien 12 de l'élément fémoral 1. L'élément 24 bute contre la surface 14 dans le vide intercondylien 12 de sorte que le mouvement relatif du plateau mobile 2 et de l'élément fémoral 1 reste contrôlé dans son amplitude.

Lors de la rotation de l'élément fémoral 1 sur le plateau mobile 2, les condyles 11 glissent ou coulissent dans les cuvettes condyliennes 21 avec le plot intercondylien 32 qui se déplace dans le vide intercondylien 12 qui forme une sorte de rail.

Le plateau mobile 2 peut se déplacer librement en translation sans aucune contrainte sur le plateau tibial 3, la surface inférieure 22 du plateau mobile et la surface supérieure 31 du plateau tibial étant toutes deux essentiellement, voire parfaitement planes.

Le plateau tibial présente une surface supérieure 31 destinée à coopérer avec la surface inférieure 22 du plateau mobile 2. Le plateau mobile 3 comprend à sa surface supérieure trois butées 32 permettant de limiter le mouvement en translation du plateau mobile 2. Il comprend sur sa face inférieure une tige massive en céramique d'alumine destinée à être cimentée dans l'extrémité supérieure du tibia préparé d'un patient.

En effet, le plateau mobile 2 joue ici le rôle de ménisque. Son périmètre est inférieur à celui du plateau tibial 3 de sorte qu'il puisse entrer en mouvement dans les limites de l'espace défini par les butées 32. Il peut donc glisser translation avant-arrière et/ou latérale à la surface supérieure du plateau tibial, ce qui a pour effet d'autoriser davantage de degrés de liberté dans le mouvement pour le patient et un mouvement très proche du mouvement naturel du genou. Il peut également pivoter autour de son axe longitudinal, parallèle à celui du plateau tibial.

L'élément fémoral 1, le plateau mobile 2 et le plateau tibial 3 sont tous trois réalisés en céramique Al₂O₃ conforme à la norme ISO-6474-1.

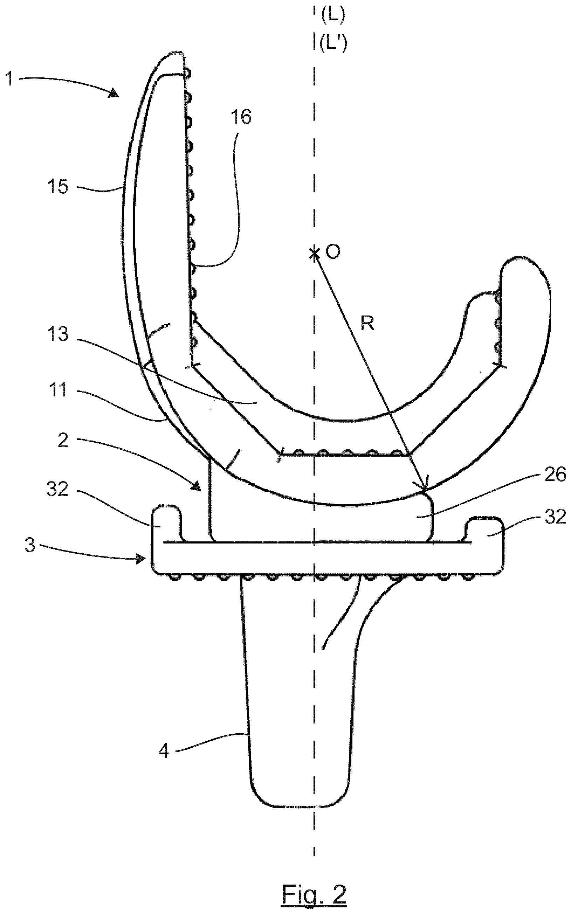

Les figures 2, 3 et 4 présentent des coupes sagittales de la prothèse assemblée selon l'invention. Les références sont identiques à celles de la figure 1. Ces coupes se font dans un plan sagittal espacé d'une distance d'environ 20-27 mm du plan médian de la prothèse.

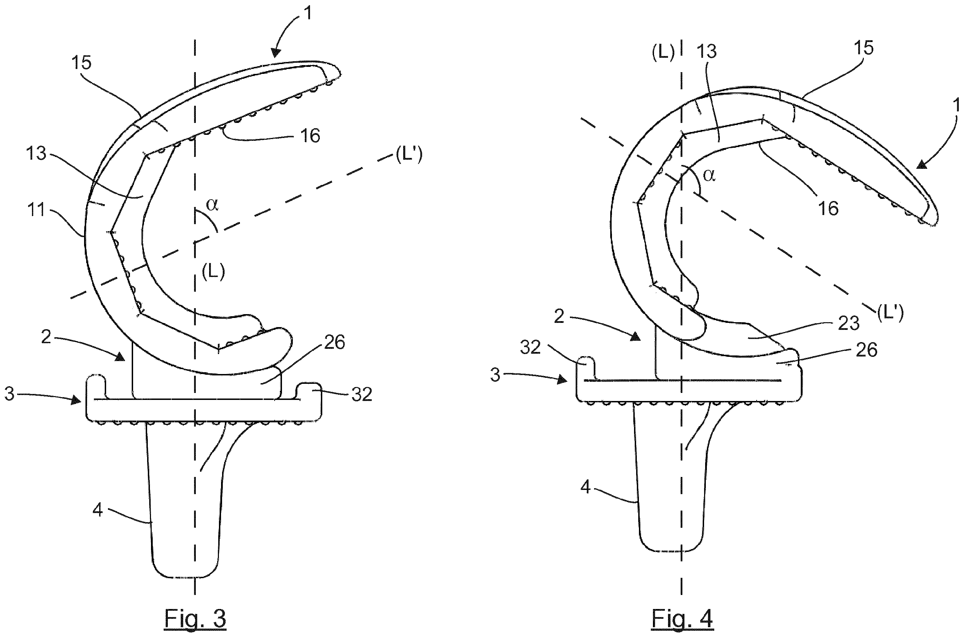

Comme on peut le voir sur la figure 2, l'axe longitudinal L du plateau tibial et l'axe longitudinal L' de l'élément fémoral forment ici un angle nul (0°). Sur la figure 3, les axes L et L' forment un angle α d'environ 45°, ce qui correspond approximativement à l'angle formé par le genou lors de la marche. Sur la figure 4, l'angle α formé par les axes L et L' est d'environ 135°, ce qui permet au patient de monter les escaliers ou pratiquer une activité sportive modérée.

Comme on peut également le voir sur la figure 2, les condyles 11 sont formés par des portions de cylindre de rayon R et dont l'axe de révolution coupe les axes L et L' au niveau du centre O. Il est à noter que les condyles pourraient être des portions de sphère de centre O et de rayon R'. De préférence, les rayons R et R' ont une longueur comprise entre 22 mm et 38 mm, de préférence entre 25 mm et 35 mm. Le rayon R" du vide intercondylien ne peut être représenté sur ces coupes, la surface du vide étant ici cachée.

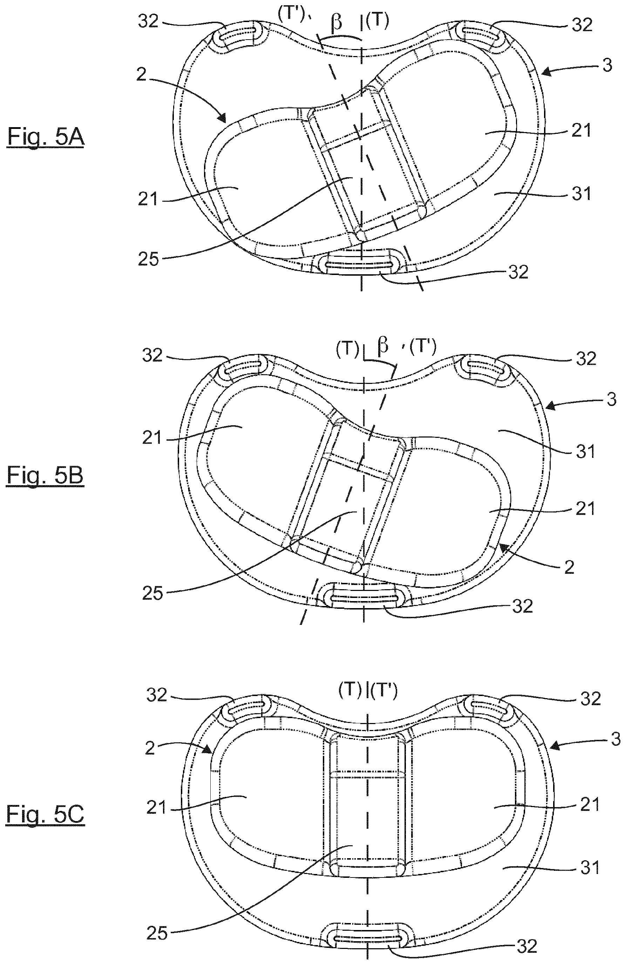

De la même manière, les figures 5A-C présentent une vue de dessus du mouvement relatif du plateau mobile 2 sur le plateau tibial 3. Comme on peut le constater, le périmètre du plateau 2 est inférieur à celui du plateau 3 de sorte que le plateau mobile 2 peut se déplacer à la surface du plateau 3. Les trois butées 32 définissent un périmètre ou champ de déplacement du plateau mobile 2. Ces vues de dessus sont destinées à illustrer l'amplitude du mouvement autorisé du plateau mobile par rapport au plateau tibial.

Comme on peut le voir sur les figures 5A et 5B, le plateau 2 peut pivoter autour de son axe longitudinal, parallèle à celui du plateau tibial (non représentés sur les figures). Plus exactement, l'axe transversal T du plateau tibial 3 peut former un angle β d'environ 15° par rapport à l'axe transversal T' du plateau mobile 2.

Par convention, on note un angle de + 15° lorsque la rotation se fait dans le sens des aiguilles d'une montre (figure 5B) et -15° lorsque la rotation est antihoraire (figure 5C).

### 7. Variantes

Différentes variantes de l'invention peuvent être envisagées. Par exemple, les condyles et cuvettes condyliennes peuvent avoir tous deux la forme d'une portion de cylindre tandis que le vide et le plot intercondyliens prennent la forme d'une portion de sphère. A l'inverse, les condyles et cuvettes condyliennes peuvent avoir tous deux la forme d'une portion de sphère tandis que le vide et le plot intercondyliens prennent la forme d'une portion de cylindre. Il est également possible qu'un condyle et sa cuvette condylienne correspondante prennent la forme d'une portion de sphère tandis que l'autre aura la forme d'une portion de cylindre.

Dans une autre variante, compatible avec les variantes ci-dessus énumérées, le plateau tibial est dépourvu de butées. En ce cas, la jambe du patient opéré est immobilisée par une attelle le temps qu'un tissu fibreux soit formé autour de la prothèse pour stabiliser l'articulation remplacée.

## Revendications

1. Prothèse de genou totale destinée à être implantée chez un patient humain, ladite prothèse comprenant un élément fémoral (1) ayant un axe longitudinal (L'), un plateau tibial (3) ayant un axe longitudinal (L) et un plateau mobile (2), ledit plateau mobile (2) étant intercalé entre l'élément fémoral (1) et le plateau tibial (3) pour former avec eux deux articulations, dans laquelle :
a. les surfaces de frottements mutuels de l'élément fémoral (1) avec le plateau mobile (2), et les surfaces de frottements mutuels du plateau tibial (3) avec le plateau mobile (2) sont constituées en un matériau céramique massif; et
b. le plateau mobile (2) comprend deux cuvettes condyliennes (21) et l'élément fémoral (1) comprend deux condyles (11), lesdits condyles (11) et lesdites cuvettes condyliennes (21) comprenant chacun des surfaces de frottements mutuels espacées l'une de l'autre d'une distance inférieure à 100 µm lorsque les axes longitudinaux (L, L') dudit élément fémoral et dudit plateau tibial forment un angle compris entre 0° et 75° ;
**caractérisée** en ce lesdits élément fémoral, plateau mobile et plateau tibial sont entièrement constitués dans le même matériau céramique, lesdits élément fémoral, plateau mobile et plateau tibial étant tous les trois réalisés en céramique massive ne comprenant pas d'alliage avec un matériau plastique ou métallique.

2. Prothèse selon l'une quelconque des revendications précédentes dans laquelle lesdites cuvettes condyliennes (21) et lesdits condyles (11) sont espacées l'une de l'autre d'une distance inférieure à 100 µm lorsque les axes longitudinaux (L, L') dudit élément fémoral et dudit plateau tibial forment un angle compris entre 0° et 60°.

3. Prothèse selon l'une quelconque des revendications précédentes dans lequel l'élément fémoral (1) et le plateau tibial (3) sont mobiles en rotation l'un part rapport à l'autre sur une plage angulaire de flexion comprise entre 0° et 135°.

4. Prothèse selon l'une quelconque des revendications précédentes dans laquelle les surfaces de frottements mutuels des condyles (11) et des cuvettes condyliennes (21) sont des portions de cylindre de révolution ayant même rayon R.

5. Prothèse selon l'une quelconque des revendications 1 à 3 dans laquelle les surfaces de frottements mutuels des condyles (11) et des cuvettes condyliennes (21) sont des portions de sphère ayant même rayon R'.

6. Prothèse selon l'une des revendications 1 à 3 dans laquelle l'une des surfaces de frottements mutuels des condyles (11) et des cuvettes condyliennes (21) est une portion de sphère ayant un rayon R' et l'autre est une portion de cylindre de révolution ayant un rayon R, le centre de la sphère étant sur l'axe de révolution du cylindre.

7. Prothèse selon l'une quelconque des revendications précédentes dans laquelle ledit plateau mobile (2) comprend un plot intercondylien (24) formant saillie en direction dudit élément fémoral (1), ledit l'élément fémoral comprenant un vide intercondylien (12) logeant ledit plot intercondylien.

8. Prothèse selon l'une des revendications 4 à 7 dans laquelle R et R' ont une longueur comprise entre 22 mm et 38 mm, de préférence entre 25 mm et 35 mm.

9. Prothèse selon l'une quelconque des revendications précédentes dans laquelle ledit plateau mobile (2) présente un périmètre inférieur au périmètre de la surface supérieure du plateau tibial (3).

10. Prothèse selon l'une quelconque des revendications précédentes dans laquelle ledit plateau tibial (3) comprend au moins trois butées (32) formant saillie en direction dudit plateau mobile (2), lesdites butées (32) permettant de limiter les mouvements du plateau mobile (2) à la surface du plateau tibial (3).

11. Prothèse selon l'une quelconque des revendications précédentes dans laquelle la surface de frottements mutuels entre ledit plateau mobile (2) et ledit plateau tibial (3) est essentiellement plane.

12. Prothèse selon l'une quelconque des revendications précédentes dans laquelle ledit plateau mobile (2) peut pivoter sur ledit plateau tibial (3) autour d'un axe selon un angle de +/-15°.

13. Prothèse selon l'une quelconque des revendications précédentes dans laquelle l'espace compris entre les surfaces de frottements mutuels du plateau mobile (2) et du plateau tibial (3) est inférieure à 100 µm.

14. Prothèse selon l'une quelconque des revendications précédentes dans laquelle ledit matériau céramique présente une épaisseur comprise entre 4 mm et 14 mm.

15. Prothèse selon l'une quelconque des revendications précédentes dans laquelle ledit matériau céramique est l'alumine Al₂O₃.

## Patentansprüche

1. Totalknieprothese zur Implantation in einen menschlichen Patienten, wobei die Prothese eine Femurkomponente (1) mit einer Längsachse (L') ein Tibiaplateau (3) mit einer Längsachse (L) und ein bewegliches Plateau (2) aufweist, wobei das bewegliche Plateau (2) zwischen der Femurkomponente (1) und dem Tibiaplateau (3) angeordnet ist, um mit jenen zwei Gelenke zu bilden, wobei:
a. die gegenseitigen Reibungsflächen der Femurkomponente (1) mit dem beweglichen Plateau (2), und die gegenseitigen Reibungsflächen des Tibiaplateaus (3) mit dem beweglichen Plateau (2) aus einem massiven keramischen Material bestehen; und
b. das bewegliche Plateau (2) zwei Kondylenschalen (21) aufweist und die Femurkomponente (1) zwei Kondylen (11) aufweist, wobei die Kondylen (11) und die Kondylenschalen (21) jeweils Reibungsflächen aufweisen, die in einem Abstand von weniger als 100 µm voneinander beabstandet sind, wenn die Längsachsen (L, L') der Femurkomponente und des Tibiaplateaus einen Winkel im Bereich zwischen 0° und 75° bilden:
**dadurch gekennzeichnet, dass** Femurkomponente, das bewegliche Plateau und das Tibiaplateau vollständig aus dem gleichen keramischen Material bestehen,
wobei Femurkomponente, bewegliches Plateau und Tibiaplateau alle drei aus massiver Keramik hergestellt sind, die keine Legierung mit einem Kunststoff- oder Metallmaterial aufweist.

2. Prothese nach einem der vorhergehenden Ansprüche, wobei die Kondylen (21) und die Kondylenschalen (11) voneinander in einem Abstand von weniger als 100 µm beabstandet sind, wenn die Längsachsen (L, L') der Femurkomponente und des Tibiaplateaus einen Winkel im Bereich zwischen 0° und 60° bilden.

3. Prothese nach einem der vorhergehenden Ansprüche, wobei die Femurkomponente (1) und das Tibiaplateau (3) über einen Beugewinkelbereich zwischen 0° und 135° relativ zueinander drehbar sind.

4. Prothese nach einem der vorhergehenden Ansprüche, wobei die gegenseitigen Reibungsflächen der Kondylen (11) und der Kondylenschalen (21) Drehzylinderabschnitte sind, die den gleichen Radius R aufweisen.

5. Prothese nach einem der Ansprüche 1 bis 3, wobei die gegenseitigen Reibungsflächen der Kondylen (11) und der Kondylenschalen (21) Kugelabschnitte sind, die den gleichen Radius R' aufweisen.

6. Prothese nach einem der Ansprüche 1 bis 3, wobei eine der gegenseitigen Reibungsflächen der Kondylen (11) und der Kondylenschalen (21) ein Kugelabschnitt ist, der einen Radius R' aufweist, und die andere ein Drehzylinderabschnitt ist, der einen Radius R aufweist, wobei sich der Mittelpunkt der Kugel auf der Drehachse des Zylinders befindet.

7. Prothese nach einem der vorhergehenden Ansprüche, wobei das bewegliche Plateau (2) einen Interkondylarblock (24) aufweist, der einen Vorsprung zur Femurkomponente (1) hin bildet, wobei die Femurkomponente einen Interkondylarspalt (12) aufweist, der den Interkondylarblock aufnimmt.

8. Prothese nach einem der Ansprüche 4 bis 7, wobei R und R' eine Länge zwischen 22 mm und 38 mm, vorzugsweise zwischen 25 mm und 35 mm haben.

9. Prothese nach einem der vorhergehenden Ansprüche, wobei das bewegliche Plateau (2) einen Umfang aufweist, der kleiner ist als der Umfang der oberen Oberfläche des Tibiaplateaus (3).

10. Prothese nach einem der vorhergehenden Ansprüche, wobei das Tibiaplateau (3) mindestens drei Anschläge (32) aufweist, die einen Vorsprung in Richtung des beweglichen Plateaus (2) bilden, wobei die Anschläge (32) es ermöglichen, die Bewegungen des beweglichen Plateaus (2) auf der Oberfläche des Tibiaplateaus (3) zu begrenzen.

11. Prothese nach einem der vorhergehenden Ansprüche, wobei die gegenseitige Reibungsfläche zwischen dem beweglichen Plateau (2) und dem Tibiaplateau (3) im Wesentlichen eben ist.

12. Prothese nach einem der vorhergehenden Ansprüche, wobei das bewegliche Plateau (2) auf dem Tibiaplateau (3) um eine Achse in einem Winkel von +/-15° schwenkbar ist

13. Prothese nach einem der vorhergehenden Ansprüche, wobei der Abstand zwischen den gegenseitigen Reibungsflächen des beweglichen Plateaus (2) und des Tibiaplateaus (3) kleiner als 100 µm ist.

14. Prothese nach einem der vorhergehenden Ansprüche, wobei das keramische Material eine Dicke von 4 mm bis 14 mm aufweist.

15. Prothese nach einem der vorhergehenden Ansprüche, wobei das keramische Material Aluminiumoxid Al₂O₃ ist.

## Claims

1. Total knee prosthesis to be implanted in a human patient, said prosthesis comprising a femoral element (1) having a longitudinal axis (L'), a tibial plateau (3) having a longitudinal axis (L) and a mobile plate (2), said mobile plate (2) being interposed between the femoral element (1) and the tibial plateau (3) to form two joints with them wherein:
a. the surfaces of mutual friction of the femoral element (1) with the mobile plate (2) and the surfaces of mutual friction of the tibial plateau (3) with the mobile plate (2) are constituted by one and the same massive ceramic material; and
b. the mobile plate (2) comprises two condylar bowls (21), and the femoral element (1) comprises two condyles (11), said condyles (11) and said condylar bowls (21) each comprising surfaces of mutual friction spaced apart from each other by a distance smaller than 100 µm when the longitudinal axes (L, L') of said femoral element and said tibial plateau form an angle of 0° to 75°,
**characterized in that** the femoral element, the mobile plate and the tibial plateau are entirely constituted out of a same ceramic material,
the femoral element, the mobile plate and the tibial plateau being all three made of massive ceramic and do not include any alloy with plastic material or metal material.

2. Prosthesis according to any one of the above claims wherein said condylar bowls (21) and said condyles (11) are spaced apart from one another by a distance of less than 100 µm when the longitudinal axes (L, L') of said femoral element and of said tibial plateau form an angle of 0° to 60°.

3. Prosthesis according to any one of the above claims wherein the femoral element (1) and the tibial plateau (3) are mobile in rotation relative to each other on an angular range of flexion of 0° to 135°.

4. Prosthesis according to any one of the above claims wherein the surfaces of mutual friction of the condyles (11) and of the condylar bowls (21) are cylinder portions generated by revolution having a same radius R.

5. Prosthesis according to any one of the claims 1 to 3 wherein the surfaces of mutual friction of the condyles (11) and the condylar bowls (21) are sphere portions having a same radius R.

6. Prosthesis according to one of the claims 1 to 3 wherein one of the surfaces of mutual friction of the condyles (11) and of the condylar bowls (21) is a sphere portion having a radius R' and the other one is a cylinder portion generated by revolution having a radius R, the center of the sphere being on the axis of revolution of the cylinder.

7. Prosthesis according to any one of the above claims wherein said mobile plate (2) comprises an intercondylar stud (24) forming a projection towards said femoral element (1), said femoral element comprising an intercondylar gap (24) that houses said intercondylar stud.

8. Prosthesis according to one of the claim 4 to 7 wherein R and R' have a length of 22 mm to 38 mm, preferably 25 mm to 35 mm.

9. Prosthesis according to any one of the above claims wherein said mobile plate (2) has a perimeter smaller than the perimeter of the upper surface of the tibial plateau (3).

10. Prosthesis according to any one of the above claims wherein said tibial plateau (3) comprises at least three stops (32) forming a projection towards said mobile plate (2), said stops (32) making it possible to limit the movements of the mobile plate (2) on the surface of the tibial plateau (3).

11. Prosthesis according to any one of the above claims wherein the surface of mutual friction between said mobile plate (2) and said tibial plateau (3) is essentially plane.

12. Prosthesis according to any one of the above claims wherein said mobile plate (2) can pivot on said tibial plateau (3) about an axis at an angle of +/- 15°.

13. Prosthesis according to any one of the above claims wherein the space between the surfaces of mutual friction of the mobile plate (2) and of the tibial plateau (3) is smaller than 100 µm

14. Prosthesis according to any one of the above claims wherein said ceramic material has a thickness of 4 mm to 14 mm.

15. Prosthesis according to any one of the above claims wherein said ceramic material is alumina Al₂O₃l₂O₃.
16. The invention relates to a total knee prosthesis to be implanted in a human patient, said prosthesis comprising a femoral element (1) having a longitudinal axis (L'), a tibial plateau (3) having a longitudinal axis (L) and a mobile plate (2), said mobile plate (2) being interposed between the femoral element (1) and the tibial plateau (3) to form two joints with them wherein:
a. the surfaces of mutual friction of the femoral element (1) with the mobile plate (2) and the surfaces of mutual friction of the tibial plateau (3) with the mobile plate (2) are entirely constituted by one and the same massive ceramic material; and
b. the mobile plate (2) comprises two condylar bowls (21), and the femoral element (1) comprises two condyles (11), said condyles (11) and said condylar bowls (21) each comprising surfaces of mutual friction spaced apart from each other by a distance smaller than 100 µm when the longitudinal axes (L, L') of said femoral element and said tibial plateau form an angle of 0° to 75°.
